# FASCICULE DE BREVET EUROPEEN

(11) **EP 3 922 894 B1**
(45) Date de publication et mention de la délivrance du brevet: **15.03.2023**
(21) Numéro de dépôt: 21168445.1
(22) Date de dépôt: 14.04.2021
(51) Int. Cl.: F16L 29/02, A61M 39/26, B01D 29/13, F16L 37/42

(54) **GUIDE-EMBOUT DE PRISE MURALE DE DISTRIBUTION DE FLUIDE EN DEUX MATIÈRES**
WANDANSCHLUSSFÜHRUNG FÜR DIE AUSGABE VON FLUIDEN AUS ZWEI STOFFEN
GUIDING END TIP FOR A FLUID DISTRIBUTION WALL SOCKET MADE OF TWO MATERIALS

(30) Priorité: 10.06.2020 FR 2006072
(43) Date de publication de la demande: 15.12.2021
(73) Titulaire: Air Liquide Medical Systems, 92160 Antony (FR)
(72) Inventeur: CHEVEREAU, Christophe, 92160 Antony (FR); BEAUCHER, Jérôme, 92160 Antony (FR); RUDNIANYN, Philippe, 92160 Antony (FR); FAVRE REGUILLON, Loic, 92160 Antony (FR)
(74) Mandataire: Air Liquide

(56) Documents cités:
- WO-A1-2019/063134
- DE-U- 7 500 293
- FR-A1- 2 628 820
- US-A- 4 851 118

## Description

L'invention concerne un guide-embout pour prise de distribution de fluide (i.e. gaz ou vide) et une prise murale de distribution de fluide équipée d'un tel guide-embout incluant un tel élément de filtration destinée à être utilisée dans un bâtiment hospitalier ou analogue, typiquement une prise murale fixée à une paroi verticale, servant à fournir du gaz ou du vide.

Les prises de distribution de fluide sont utilisées pour distribuer les fluides, en particulier les gaz médicaux (i.e. un gaz pur ou un mélange gazeux) ou le vide (i.e. dépression < 1 atm), au sein des bâtiments hospitaliers ou analogues. Elles sont couramment appelées « prises murales » ou « raccords muraux » car elles sont généralement montées soit directement sur les parois, c'est-à-dire les murs ou analogues, des bâtiments hospitaliers, soit indirectement, par exemple en étant intégrées à un boitier ou analogue qui est lui-même monté sur une paroi, en particulier dans les chambres des patients, dans les salles d'opération ou de soins, notamment les salles de réanimation, ou d'autres pièces

Les prises murales permettent de fournir les fluides médicaux, c'est-à-dire les gaz médicaux véhiculés par les réseaux de canalisations de gaz parcourant les bâtiments hospitaliers, aux appareils et équipements utilisés pour traiter et soigner les patients au sein de ces bâtiments, en particulier les gaz thérapeutiques, tel que l'oxygène, le protoxyde d'azote ou l'air, ou le vide médical (i.e. dépression) permettant d'opérer des aspirations notamment de liquides biologiques, par exemple le sang ou d'autres liquides biologiques.

Ainsi, le document FR-A-2628820 ou DE7500293U proposent une prise de distribution de fluide, appelée raccord à verrouillage automatique, ayant une architecture classique.

Elle comprend un corps de prise de forme allongée comprenant une cavité ou passage central traversant axialement le corps de prise de sorte de relier fluidiquement une extrémité amont, aussi appelée extrémité d'entrée ou extrémité distale, à une extrémité aval, aussi appelée extrémité de sortie ou extrémité proximale comprenant un orifice de fourniture de fluide, c'est-à-dire de gaz ou de vide. Le fluide « circule » dans la prise, lorsqu'un connecteur d'un appareil ou équipement médical, notamment le connecteur d'une conduite de gaz, est raccordé mécaniquement et fluidiquement à la prise.

Des éléments de contrôle du passage de fluide internes à la prise murale permettent de contrôler la libération du gaz, en particulier d'empêcher toute délivrance du gaz quand aucun connecteur s'y est branché. Ces éléments de contrôle du passage de fluide comprennent un guide-embout extractible agencé du côté proximal de la prise, c'est-à-dire vers l'avant de la prise, et une chambre à bille-clapet extractible, du côté distal de la prise, c'est-à-dire vers l'arrière ou le fond de la prise, qui sert à empêcher toute circulation de fluide lorsque le guide-embout est extrait du corps de prise, ou à limiter le débit de fuite.

Le guide-embout comprend un clapet de tête coulissant et l'orifice de sortie de gaz ou d'aspiration pour le vide. Le clapet de tête coulissant coopère avec un siège de clapet pour assurer ou, à l'inverse, interrompre l'étanchéité fluidique entre eux et ainsi empêcher ou, à l'inverse, autoriser le passage de fluide, i.e. gaz ou vide, lorsqu'un connecteur d'un appareil ou équipement médical est raccordé à la prise murale. Un élément élastique, tel un ressort, agit sur le clapet de tête coulissant pour le repousser en direction du siège de clapet.

Le guide-embout comprend ou est formé habituellement deux parties, à savoir une partie arrière et une partie avant, fixées l'une à l'autre, par exemple par vissage, lesquelles sont typiquement en métal, par exemple en acier inoxydable.

La partie avant ou « tête » du guide-embout comprend des moyens de fixation dudit guide-embout au sein de la prise de distribution de fluide, par exemple un filetage périphérique externe, et est par ailleurs traversé par un passage axial en communication fluidique avec l'orifice de la partie arrière du guide-embout qui communique fluidiquement avec le logement interne de la partie arrière du guide-embout.

La partie arrière ou « corps » de guide-embout comprend une paroi périphérique délimitant un logement interne, i.e. un volume interne ou lumen, s'étendant entre une extrémité ouverte et une extrémité borgne. L'extrémité ouverte porte un orifice communiquant avec le logement interne, alors que la paroi périphérique comprenant une ou plusieurs ouvertures latérales de passage de gaz communiquant avec le logement interne. Le fluide circule au travers des ouvertures latérales et/ou de l'orifice pour entrer ou sortir du logement interne. Le clapet de tête coulissant est agencé mobile dans le logement interne.

Ce type de prise murale doit subir régulièrement des opérations de maintenance, notamment afin de garantir leur bon fonctionnement, de remplacer les éléments usés ou détériorés (e.g. utilisation intensive), de les nettoyer.... Lors de ces opérations, il faut pouvoir démonter puis remonter facilement et rapidement les différents éléments internes de la prise murale, en particulier le guide-embout situé vers l'avant du corps de prise et la chambre à bille-clapet. Il doit aussi être possible de remplacer uniquement les éléments usés ou détériorés du guide-embout, sans qu'il ne soit besoin de les remplacer tous, en particulier ceux qui ne sont pas usés ou détériorés.

Enfin, il faut également assurer une filtration du fluide (i.e. gaz ou vide) traversant la prise murale afin de garantir qu'il soit exempt de poussières ou autres contaminants/polluants solides.

Un problème est de pouvoir améliorer la structure du guide-embout d'une prise de distribution de fluide (i.e. gaz ou vide), en particulier la partie arrière du guide-embout, de manière à pouvoir répondre aux différentes exigences et/ou problématiques susmentionnées. De plus, un autre problème concerne la fabrication du guide-embout qui génère actuellement beaucoup de déchets métalliques, sachant que le guide-embout doit par ailleurs être robuste pour résister aux opérations de connexion/déconnexion fréquentes de tuyaux flexibles ou d'appareils ou dispositifs médicaux à la prise de distribution de fluide intégrant le guide-embout.

La solution concerne alors un guide-embout pour prise de distribution de fluide, i.e. gaz ou vide, comprenant une partie avant et une partie arrière solidarisées l'une à l'autre, dans lequel :
- la partie arrière comprend un élément de filtration comprenant une pièce-support comprenant une paroi périphérique délimitant un logement interne et s'étendant entre une extrémité ouverte comprenant un orifice et une extrémité borgne, l'orifice communiquant avec le logement interne, et la paroi périphérique comprenant en outre au moins une ouverture latérale communiquant avec le logement interne, au moins une membrane de filtration de gaz étant agencée dans ladite au moins une ouverture latérale de manière à recouvrir ladite au moins une ouverture latérale, et
- la partie avant comprend une tête de raccordement comprenant un passage axial de gaz en communication fluidique avec le logement interne de l'élément de filtration de gaz, et des moyens de fixation configurés pour permettre une fixation détachable du guide-embout au sein d'une prise de distribution de fluide, et
- la partie avant et la partie arrière comprennent en outre des moyens de solidarisation réciproques permettant d'assurer une fixation détachable desdites partie avant et partie arrière, l'une à l'autre,
caractérisé en ce que :
- la pièce-support de l'élément de filtration de la partie arrière est en matériau polymère, et
- la tête de raccordement de la partie avant est en matériau métallique.

La solution de l'invention résout les problèmes susmentionnés étant donné qu'un guide-embout en deux matériaux différents, à savoir formé :
- d'un élément de filtration formé en tout ou en partie de polymère permet de réduire la quantité de déchets métalliques générés pendant sa fabrication et permet une fabrication plus simple de cet élément, par exemple par (sur)moulage,
- et d'une tête de raccordement en métal permet d'offrir une robustesse suffisante à l'ensemble et d'assurer une connexion efficace aux tuyaux flexibles ou autres appareils ou dispositifs médicaux qui seront raccordés à la prise de distribution de fluide intégrant ce guide-embout.

Selon le mode de réalisation considéré, le guide-embout de l'invention peut comprendre l'une ou plusieurs des caractéristiques suivantes :
- les moyens de fixation de la partie avant du guide-embout sont configurés pour permettre une fixation détachable du guide-embout au sein d'une prise de distribution de fluide comprennent un filetage périphérique externe destiné à venir coopérer avec un taraudage aménagé dans le corps de la prise distribution de fluide.
- la pièce-support de l'élément de filtration de la partie arrière est en polyamide.
- les moyens de solidarisation réciproques comprennent un taraudage et un filetage coopérant l'un avec l'autre, le filetage étant agencé dans la partie avant et le taraudage étant agencé dans la partie arrière, ou inversement.
- les moyens de fixation de la partie avant du guide-embout configurés pour permettre une fixation détachable du guide-embout au sein d'une prise de distribution de fluide comprennent un filetage périphérique externe destiné à venir coopérer avec un taraudage aménagé dans le corps de la prise distribution de fluide.
- la tête de raccordement de la partie avant est en acier inoxydable ou en laiton, de préférence en acier inoxydable ou en laiton revêtu d'un revêtement métallique externe, notamment de chrome ou de nickel.
- la tête de raccordement de la partie avant est (au moins en partie) cylindrique.
- la pièce-support a une forme générale tubulaire à fond borgne, c'est-à-dire fermée à une extrémité.
- l'extrémité ouverte de la pièce-support comprend un orifice au travers duquel le fluide (i.e. gaz ou vide) peut circuler, c'est-à-dire entrer ou sortir de l'élément de filtration.
- le logement interne de la pièce-support a une forme cylindrique.
- la pièce-support comprend plusieurs ouvertures latérales, chaque ouverture latérale comprenant une membrane de filtration de gaz.
- la pièce-support comprend de 2 à 6 ouvertures latérales, de préférence de 2 à 4 ouvertures latérales.
- les ouvertures latérales sont préférentiellement identiques les unes aux autres ou, selon un autre mode de réalisation, elles peuvent avoir des formes différentes les uns des autres.
- les membranes de filtration recouvrent complètement les ouvertures latérales dans lesquelles elles sont agencées, c'est-à-dire que les sections de passage des ouvertures latérales sont totalement obturées par les membranes de filtration.
- les membranes de filtration sont conçues pour filtrer le fluide (i.e. gaz ou vide) qui les traversent.
- les membranes de filtration sont formées d'une pièce unique de forme cylindrique.
- la pièce cylindrique portant les membranes de filtration a un diamètre inférieur ou égal, de préférence approximativement égal, au diamètre interne du logement interne de la pièce-support.
- selon un mode de réalisation, la pièce cylindrique portant les membranes de filtration est insérée et non fixée (i.e. libre) dans le logement interne de la pièce-support.
- selon un autre mode de réalisation, la pièce cylindrique portant les membranes de filtration est insérée et fixée (i.e. libre) dans le logement interne de la pièce-support.
- les membranes de filtration sont fixées à la pièce-support par collage, thermo-soudage, surmoulage ou autre, de préférence par surmoulage de la pièce-support.
- les membranes de filtration sont formées d'une toile ou tissu en polymère.
- les membranes de filtration comprennent des pores compris entre 50 et 80 µm, en particulier lorsqu'elles sont destinées à filtrer un gaz, notamment un gaz médical.
- selon un autre mode de réalisation, les membranes de filtration comprennent des pores compris entre 200 et 300 µm, en particulier lorsqu'elles sont destinées à filtrer du vide ou de l'air-instrument.
- les membranes de filtration sont formées d'une pièce unique de forme cylindrique formée d'une toile ou d'un tissu en polymère.
- la pièce-support est surmoulée autour de la pièce unique de forme cylindrique formée d'une toile ou d'un tissu en polymère.
- les membranes de filtration sont formées d'une pièce en polymère de forme cylindrique autour de laquelle est surmoulée la pièce-support.
- la pièce-support comprend un axe longitudinal (XX), c'est-à-dire qu'elle s'étend axialement selon l'axe longitudinal (XX).
- la pièce-support est une pièce de révolution.
- chaque membrane de filtration a une épaisseur inférieure à l'épaisseur de la paroi périphérique autour de l'ouvertures latérale dans laquelle elle est agencée.
- les membranes de filtration sont préférentiellement en polymère, par exemple en polyamide, tel du Nylon^{®}.
- les membranes de filtration sont, selon un autre mode de réalisation, en métal, par exemple de l'acier inoxydable.
- la pièce-support forme une armature, i.e. un châssis ou cadre, portant les membranes de filtration.
- l'armature constituant la pièce-support est formée d'une pièce en polymère de type polyamide.
- la pièce-support a une forme générale cylindrique.
- les ouvertures latérales sont de section carrée ou rectangulaire, ou autre, par exemple ovale ou ellipsoïdale.
- la pièce-support comprend plusieurs tronçons successifs de diamètres externes différents, i.e. des tronçons cylindriques ou approximativement cylindriques.
- la pièce-support comprend un tronçon amont à son extrémité ouverte ; un tronçon aval à son extrémité borgne ; et au moins un tronçon intermédiaire agencé entre les tronçons amont et aval, ledit tronçon intermédiaire comprenant les ouvertures.
- les tronçons amont et aval sont cylindriques.
- le tronçon intermédiaire est cylindrique ou approximativement cylindrique.
- le diamètre externe du tronçon intermédiaire comprenant les ouvertures est inférieur aux diamètres externes des tronçons amont et aval.
- les tronçons amont et aval sont reliés l'un à l'autre par des éléments de paroi longilignes formant tout ou partie du tronçon intermédiaire.
- les éléments de paroi longilignes sont parallèles les uns aux autres et à l'axe longitudinal (XX).
- les ouvertures du tronçon intermédiaire sont bordées par les tronçons amont et aval, et les éléments de paroi longilignes.
- les tronçons amont et aval, et les éléments de paroi longilignes sont formés d'une seule pièce, par exemple par moulage.
- les tronçons amont et aval, et les éléments de paroi longilignes sont formés de matériau polymère, i.e. sont en plastique, typiquement en polyamide.
- les tronçons amont et aval, et les éléments de paroi longilignes de la pièce-support forme l'armature, i.e. le châssis ou cadre, portant les membranes de filtration.
- le diamètre externe du tronçon amont est par exemple compris entre 12 et 16 mm.
- le diamètre externe du tronçon aval est par exemple compris entre 11 et 15 mm.
- le diamètre externe du tronçon cylindrique intermédiaire est par exemple compris entre 11 et 15 mm.
- l'extrémité borgne de la pièce-support comprend une tige-poussoir faisant saillie axialement (axe XX) au centre de la face externe de l'extrémité borgne.
- la pièce-support a une longueur de l'ordre de 20 à 25 mm.
- le logement interne de la pièce-support a un diamètre interne de l'ordre de 9 à 12 mm.
- l'extrémité ouverte de la pièce-support comprend un taraudage permettant de la fixer à la partie avant d'un guide-embout.
- un clapet de tête coulissant est agencé dans le logement interne de l'élément de filtration.
- un moyen élastique, tel un ressort, est agencé dans le logement interne de l'élément de filtration.
- le moyen élastique est agencé pour agir sur, i.e. repousser, le clapet de tête en direction d'un siège de clapet.
- la partie avant du guide-embout comprend une gorge périphérique annulaire et un élément de joint agencé dans ladite gorge périphérique annulaire, en particulier un joint torique.
- la partie avant du guide-embout comprend le siège de clapet coopérant avec le clapet de tête.

L'invention concerne aussi une prise de distribution de fluide, c'est-à-dire de gaz ou de vide (i.e. dépression), comprenant un corps de prise dans lequel est agencé un élément de filtration de gaz selon l'invention.

Selon le mode de réalisation considéré, la prise de distribution de fluide de l'invention peut comprendre l'une ou plusieurs des caractéristiques suivantes :
- le corps de prise comprend en outre une chambre à bille contenant une bille-clapet coopérant avec la tige-poussoir de la pièce-support du guide-embout.
- la chambre à bille est extractible.
- la chambre à bille est visée dans le corps de prise, en particulier dans le passage central du corps de prise.
- elle est une prise murale, c'est-à-dire qu'elle comprend des moyens de fixation permettant de la fixer à une paroi, tel un mur ou analogue.
- le guide-embout est monté de manière amovible, c'est-à-dire démontable, dans le corps de prise.
- elle est reliée à une canalisation de gaz ou de vide, en particulier au réseau de canalisations de gaz ou de vide (i.e. dépression) d'un bâtiment hospitalier.

L'invention concerne en outre une utilisation d'une prise de distribution de fluide selon l'invention pour distribuer, i.e. fournir, un gaz ou mélange gazeux, ou du vide (i.e. aspiration à une pression < 1 atm) au sein d'un bâtiment hospitalier.

Le gaz ou mélange gazeux peut être de l'oxygène, de l'air, un mélange N₂O/O₂.... ou autre.

La prise de distribution de fluide selon l'invention est reliée fluidiquement au réseau de canalisation du bâtiment hospitalier.

L'invention va maintenant être mieux comprise grâce à la description détaillée suivante, faite à titre illustratif mais non limitatif, en référence aux figures annexées parmi lesquelles :
Fig. 1 est une vue latérale d'un élément de filtration de gaz formant la partie arrière d'un guide-embout de prise de distribution de fluide selon l'invention,
Fig. 2 est une vue latérale schématique d'un guide-embout selon l'invention, et
Fig. 3 est une vue en coupe d'une prise de distribution de gaz incluant un guide-embout selon l'invention.

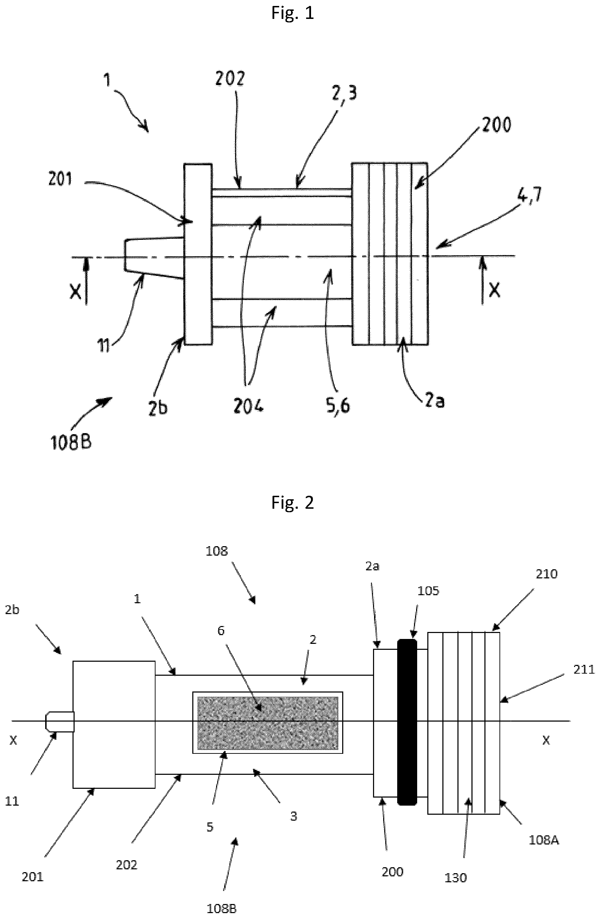

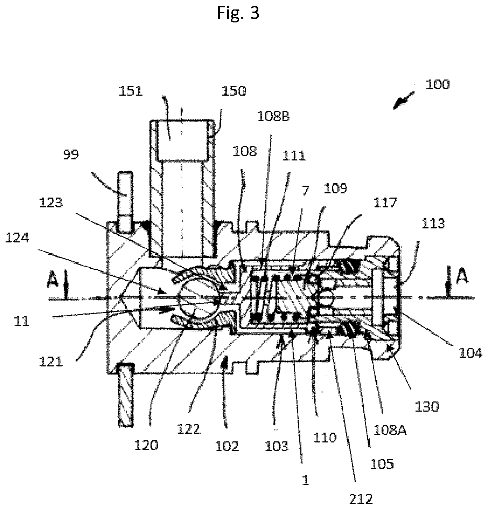

Fig. 3 représente un mode de réalisation d'une prise de distribution 100 de gaz selon l'invention, par exemple d'oxygène ou d'air médical, comprenant un corps de prise 102 de forme allongée, selon un axe longitudinal AA, lequel est traversé axialement par un passage central 103 formant un logement interne au sein duquel sont agencés des éléments de contrôle du passage de gaz, en particulier le guide-embout 108 selon l'invention illustré en Fig. 2 et une chambre à bille 122, comme expliqué ci-après. Une telle architecture est classique.

Le guide-embout 108 selon l'invention extractible est formé de deux parties principales fixées l'une à l'autre, par exemple par vissage, à savoir une partie avant 108A incluant une « tête » de raccordement 210 de guide-embout 108 et une partie arrière 108B ou « corps » de guide-embout 108 comprenant l'élément de filtration de gaz 1, comme illustré sur les Fig. 1 et Fig. 2 et détaillé ci-après. La tête 210 de raccordement de la partie avant a une forme générale cylindrique.

Selon l'invention, la pièce-support 2 de l'élément de filtration 1 de la partie arrière 108B du guide-embout 108 est en matériau polymère, de préférence en polyamide, alors que la tête de raccordement 210 de la partie avant 108A est en matériau métallique, de préférence en acier inoxydable ou en laiton, de préférence en acier inoxydable ou en laiton revêtu d'un revêtement métallique externe, notamment de chrome ou de nickel.

La partie avant 108A comprend des moyens de fixation 130 du guide-embout 108 au sein de la prise de distribution de fluide 100, à savoir ici un filetage périphérique externe schématisé sur la Fig. 2 venant coopérer avec un taraudage aménagé dans le corps 102 de prise 100, et est par ailleurs traversé par un passage axial 211 comprenant un orifice proximal 104 par lequel le gaz peut sortir du passage axial 211 de la partie avant 108A pour alimenter un tuyau flexible, un appareil ou dispositif médical ou autre, ou par lequel se fait l'aspiration de vide.

Dans le cas de la prise 100 de Fig. 3, le gaz est distribué, c'est-à-dire sort de la prise 100, par l'orifice proximal 104 lorsqu'un appareil, dispositif ou équipement utilisant ou véhiculant le gaz y est raccordé via un connecteur adapté, tel un conduit flexible amenant le gaz à un appareil de respiration assisté, tel un ventilateur médical. L'orifice proximal 104 est de section circulaire. A l'inverse, lorsque la prise 100 est une prise de vide, c'est par cet orifice proximal 104 que s'opère l'aspiration du gaz, c'est-à-dire que s'applique la dépression, permettent d'aspirer les liquides biologiques ou autres.

Par ailleurs, la partie arrière 108B du guide-embout 108 comprend l'élément de filtration de gaz 1 selon l'invention, comme illustré en Fig. 2.

Comme détaillé en Fig. 1 et Fig. 2, l'élément de filtration de gaz 1 formant tout ou partie de la partie arrière 108B du guide-embout 108 de l'invention, comprend une pièce-support 2 avec une paroi périphérique 3 délimitant un logement interne 7 ou lumen. La pièce-support 2 est de forme allongée, c'est-à-dire tubulaire. Elle s'étend entre une extrémité ouverte 2a comprenant un orifice 4 et une extrémité borgne 2b. La pièce-support 2 forme une armature, c'est-à-dire un châssis ou un cadre, portant des membranes de filtration 6, comme expliqué ci-après. Elle est formée préférentiellement d'une pièce unique en polymère de type polyamide.

L'orifice 4 de l'élément de filtration de gaz 1 communique avec le logement interne 7 de celui-ci. La paroi périphérique 3 comprenant en outre des ouvertures latérales 5, par exemple de 2 à 4 ouvertures latérales, ici de forme rectangulaire, communiquant avec le logement interne 7. Une membrane de filtration 6 de gaz est agencée dans chacune des ouvertures latérales 5 de manière à recouvrir les ouvertures latérales 5 de sorte que le gaz passant par ces ouvertures latérales 5 soit filtré par les membranes de filtration 6 qui y sont disposées. Le fluide, i.e. gaz ou vide, circule au travers des ouvertures latérales 5 et de l'orifice 4 pour entrer ou sortir du logement interne 7.

Un clapet de tête 109 coulissant est agencé mobile selon l'axe AA dans le logement interne 7, i.e. coulissant en translation, au sein dudit logement axial 7, comme illustré en Fig. 3. De plus, un élément élastique 111, tel un ressort cylindrique ou analogue, est agencé dans le logement axial 7 de l'élément de filtration 1 du guide-embout 108, de préférence dans le fond borne et autour de la portion arrière du clapet de tête 109, lequel élément élastique 111 vient prendre appui, d'une part, sur le fond borgne de l'élément de filtration 1 et, d'autre part, sur un épaulement annulaire solidaire du clapet de tête 109, par exemple formé dans la paroi périphérique externe du clapet de tête 109. L'élément élastique 111 permet de normalement repousser le clapet de tête 109 contre un siège de clapet 110 aménagé dans le guide-embout 108, par exemple au sein de la paroi interne du guide-embout 108, de sorte de contrôler le passage de fluide dans le corps de prise 102.

Par ailleurs, le guide-embout 108 de l'invention comprend aussi un élément d'étanchéité 105 permettant d'assurer une étanchéité fluidique entre ledit guide-embout 108 et le corps de prise 102 au sein duquel il est fixé de manière amovible, c'est-à-dire détachable.

Un autre élément d'étanchéité 117, porté par le clapet 109, permet quant à lui d'assurer une étanchéité fluidique entre le clapet 109 et le siège de clapet 110, lorsque le clapet de tête 109 est repoussé contre le siège de clapet 110 par l'élément élastique 111.

Par ailleurs, la prise 100 de distribution de fluide de Fig. 3 comprend aussi un système de sécurité agencé au fond du passage central 103, c'est-à-dire au fond du corps de prise 102, permettant d'empêcher ou limiter la circulation de gaz en direction de l'orifice de sortie 104, lorsque le guide-embout 108 est extrait du corps de prise 102, par exemple lors d'une opération de maintenance du guide-embout 108. Ce système de sécurité, appelé chambre à bille-clapet 122, comprend un compartiment 121, i.e. une chambre, dans lequel est logée une bille-clapet 120, à savoir ici une bille de forme sphérique, coopérant avec un siège de bille-clapet situé dans le compartiment 121 de la chambre à bille-clapet 122.

La chambre à bille-clapet 122 est aussi extractible du corps de prise 102 afin de permettre sa maintenance. Elle permet d'empêcher que le gaz ne puisse s'échapper de la prise 100 ou que l'aspiration par le vide (i.e. dépression) ne se fasse, c'est-à-dire que de l'air ne puisse entrer dans le réseau de vide relié à la prise 100 et y faire remonter la pression (< 1 atm) qui y règne, lorsque les éléments de contrôle du passage de gaz, typiquement le guide-embout 108, sont démontés et extraits du corps de prise 102, notamment lors d'une opération de vérification, de maintenance ou de remplacement.

La bille-clapet 120 fait office de clapet de sécurité venant, sous l'effet de la pression fluidique ou de la dépression (i.e. vide) s'exerçant sur la bille-clapet 120 en cas de démontage du guide-embout 108, appuyer sur et obturer un siège de clapet situé dans le compartiment 121. A l'inverse, quand les éléments de contrôle du passage de gaz sont montés dans le corps de prise 102, le guide-embout 108 vient appuyer sur la bille-clapet 120 pour la décoller du siège de clapet et autoriser ainsi la communication fluidique au travers du canal de liaison 123 reliant fluidiquement le compartiment 121 au passage axial 103 du corps 102 de la prise 100. Ceci peut se faire via une tige-poussoir 11, c'est-à-dire une tige ou expansion axiale, faisant saillie sur la surface externe arrière du guide-embout 8 et solidaire de celui-ci, qui est orientée de sorte de traverser axialement le canal de liaison 123 pour venir appuyer sur la bille-clapet 120 et ainsi la décoller de son siège. La tige axiale 11 est portée par la pièce-support 2 de l'élément de filtration 1 du guide-embout 8 de l'invention, comme visible sur les Fig. 1 à Fig. 3, et détaillé ci-après.

La prise de distribution 1 de fluide de l'invention, telle celle illustrée en FIG. 3, peut être montée soit directement sur une paroi (non montrée), c'est-à-dire un mur ou analogue, d'un bâtiment hospitalier au moyen d'un étrier de fixation 99, soit être intégrées à un boitier ou analogue qui est lui-même monté sur une paroi. Elle permet de fournir le vide ou les gaz médicaux véhiculés par le réseau de canalisations de fluide parcourant le bâtiment hospitalier, aux appareils et équipements utilisés pour traiter et soigner les patients au sein de ces bâtiments. Pour ce faire, elles peuvent être raccordées au réseau de canalisations de fluide d'un tel bâtiment, via une tubulure arrière 150, tel un raccord ou analogue, en communication fluidique, via son lumen 151, avec, d'une part, ledit réseau de canalisations de fluide et, d'autre part, avec le passage central interne de la prise 100 via la chambre à bille 122 qui communique avec le lumen 151 de la tubulure arrière 150, via une ouverture large 124 de la chambre à bille 122.

La partie arrière 108B du guide-embout 108 comprend l'élément de filtration 1, illustré sur la Fig. 1, lequel vient se fixer, par exemple par vissage, à la partie avant 108A du guide-embout 108, comme schématisé en Fig. 2 et Fig. 3.

Cet élément de filtration 1 comprend, comme visible sur Fig. 1 et Fig. 2, une pièce-support 2, formant un corps tubulaire allongé, comprenant plusieurs tronçons successifs 200-202 ayant des diamètres externes différents, à savoir un tronçon amont 200 à son extrémité ouverte 2a, un tronçon aval 201 à son extrémité borgne 2b, et un tronçon intermédiaire 202 agencé entre les tronçons amont et aval 200, 201, lequel porte les ouvertures 5 obturées par les membranes de filtration 6.

Les tronçons amont 200 et aval 201 sont cylindriques et ont des diamètres externes égaux ou différents, par exemple compris entre 11 et 16 mm, alors que le tronçon intermédiaire 202 est cylindrique ou approximativement cylindrique, et a un diamètre inférieur au ou aux diamètres externes des tronçons amont 200 et aval 201, par exemple compris entre 11 et 15 mm. La pièce-support 2 a par exemple une longueur de l'ordre de 20 à 25 mm.

Les ouvertures 5 sont ici de forme rectangulaire mais, bien entendu, elles pourraient être d'une autre forme, par exemple de forme carrée, ovale, ellipsoïdale ou autre. Les membranes de filtration 6 ont des formes complémentaires de celles des ouvertures 5 de manière à les recouvrir complètement de sorte que tout le gaz passant au travers des ouvertures 5 soit filtré par ces membranes de filtration 6.

Dans le mode de réalisation de la Fig. 1, les tronçons amont 200 et aval 201 sont des cylindres reliés l'un à l'autre par des éléments de paroi longilignes 203 formant tout ou partie du tronçon intermédiaire 202. Ces éléments de paroi longilignes 204 sont parallèles les uns aux autres et à l'axe longitudinal (XX). Les ouvertures 5 sont donc bordées par les tronçons amont 200 et aval 201, et les éléments de paroi longilignes 204, lesquels forment le cadre ou châssis.

Avantageusement, les tronçons amont 200 et aval 201, et les éléments de paroi longilignes 204 constituant l'élément de filtration 1 sont formés d'une seule pièce de polymère, i.e. plastique, par exemple par moulage ou analogue, typiquement un polyam ide.

Comme on le voit sur la Fig. 1, l'extrémité borgne 2b de la pièce-support 2 porte, sur face externe, la tige-poussoir 11 qui coopère avec la bille-clapet 120, comme expliqué ci-avant. La tige-poussoir 11 fait saillie axialement (selon l'axe XX) au centre de la face externe de l'extrémité borgne de la pièce-support 2.

Le logement interne 7 de la pièce-support 2 a un diamètre interne de l'ordre de 9 à 12 mm de manière à pouvoir accueillir le clapet de tête 109 et le ressort 111, comme expliqué ci-avant.

Afin de permettre de fixer l'élément de filtration 1 formant la partie arrière 108B du guide-embout 108, à la partie avant 108A du guide-embout 108, sont prévus des moyens de solidarisation réciproques 212 configurés pour assurer une fixation détachable desdites parties avant 108A et arrière 108B, l'une à l'autre, comme illustré en Fig. 3. Plus précisément, pour ce faire, l'extrémité ouverte 2a de la pièce-support 2 de l'élément de filtration 1 comprend un taraudage permettant de fixer l'élément de filtration 1 à la partie avant 108A du guide-embout 108. Un filetage réciproque est donc prévu sur la partie avant 108A du guide-embout 108 lequel coopère avec le taraudage porté par la partie arrière 108B du guide-embout 108, c'est-à-dire par la pièce-support 2 de l'élément de filtration 1. Bien entendu, un autre système de fixation adapté pourrait être utilisé en lieu et place du taraudage et du filetage, par exemple un système à baïonnette ou autre.

Les membranes de filtration 6 agencées dans les ouvertures 5 sont conçues pour filtrer le fluide, i.e. gaz ou vide, qui les traversent. Elles peuvent être en polymère ou en métal, de préférence en polymère, par exemple en polyamide. Elles ont des pores ayant des dimensions inférieures aux particules qu'elles doivent arrêter, telles des poussières, des débris, des résidus, des bactéries ou toutes autres particules, par exemple des pores de diamètre compris entre 50 et 80 µm, lorsqu'elles sont destinées à filtrer un gaz, notamment un gaz médical (air médical, oxygène...), ou compris entre 200 et 300 µm, lorsqu'elles sont destinées à filtrer du vide (i.e. dépression) ou de l'air-instrument.

Avantageusement, les membranes de filtration 6 sont constituées ou portées par une pièce unique de forme cylindrique préférentiellement formée d'une toile de polyamide, tel du Nylon^{®}, autour de laquelle est surmoulé le reste de la pièce support 2, en particulier les tronçons amont 200 et aval 201, et les éléments de paroi longilignes 204 qui forment le cadre ou châssis. De préférence, on utilise pour former les membranes 6, une toile en polyamide de référence SEFAR-NITEXO commercialisée par la société Sefar.

Fig. 3 représente un mode de réalisation d'une prise 100 de distribution de gaz dans laquelle est monté un guide-embout 108 détachable selon l'invention. Son architecture globale est très similaire à celle d'une prise de distribution de vide, c'est-à-dire de dépression. Une différence majeure réside toutefois dans le fait que, dans une prise de distribution de vide, l'aspiration du vide (i.e. dépression), c'est-à-dire la circulation du gaz aspiré au travers de la prise, se fait dans le sens opposé à celui de la circulation de gaz au sein de la prise 100 de Fig. 3. Ainsi, le gaz (e.g. air) est aspiré par la dépression régnant dans le corps de prise 102 par l'orifice proximal 104, ce qui permet d'obtenir un effet de succion, c'est-à-dire d'aspiration, en amont de la prise 100, c'est-à-dire dans un dispositif médical (non montré) raccordé fluidiquement à la prise 100. L'orifice proximal 104 sert donc à aspirer du gaz et non pas à en distribuer. Une conséquence de cette circulation inverse du fluide est que, dans une prise 100 de distribution de vide, la chambre à bille est orientée à l'inverse de celle de la Fig. 3 et que par ailleurs, la bille-clapet n'est pas complètement sphérique mais comprend par exemple uniquement une tête hémisphérique sur laquelle vient appuyer la tige 111 du guide-embout 108 au travers du canal de liaison 122, comme expliqué ci-avant.

D'une façon générale, les prises 1 de l'invention sont généralement agencées sur des parois, tels des murs ou analogues, des bâtiments hospitaliers et sont conçues pour permettre de fournir soit des gaz thérapeutiques, i.e. gaz médicaux, en particulier de l'oxygène, du protoxyde d'azote, de l'air ou tout autre gaz ou mélange gazeux, soit du vide médical (i.e. une dépression) servant à opérer des aspirations notamment de liquides biologiques, par exemple le sang ou d'autres liquides biologiques.

## Revendications

1. Guide-embout (108) pour prise de distribution de fluide (100) comprenant une partie avant (108A) et une partie arrière (108B) solidarisées l'une à l'autre, dans lequel :
- la partie arrière (108B) comprend un élément de filtration (1) comprenant une pièce-support (2) comprenant une paroi périphérique (3) délimitant un logement interne (7) et s'étendant entre une extrémité ouverte (2a) comprenant un orifice (4) et une extrémité borgne (2b), l'orifice (4) communiquant avec le logement interne (7), et la paroi périphérique (3) comprenant en outre au moins une ouverture latérale (5) communiquant avec le logement interne (7), au moins une membrane de filtration (6) de gaz étant agencée dans ladite au moins une ouverture latérale (5) de manière à recouvrir ladite au moins une ouverture latérale (5), et
- la partie avant (108A) comprend une tête de raccordement (210) comprenant un passage axial de gaz (211) en communication fluidique avec le logement interne (7) de l'élément de filtration de gaz (1), et des moyens de fixation (130) configurés pour permettre une fixation détachable du guide-embout (108) au sein d'une prise de distribution de fluide (100), et
- la partie avant (108A) et la partie arrière (108B) comprennent en outre des moyens de solidarisation (212) réciproques permettant d'assurer une fixation détachable desdites partie avant (108A) et partie arrière (108B), l'une à l'autre,
**caractérisé en ce que** :
- la pièce-support (2) de l'élément de filtration (1) de la partie arrière (108B) est en matériau polymère, et
- la tête de raccordement (210) de la partie avant (108A) est en matériau métallique.

2. Guide-embout selon la revendication 1, **caractérisé en ce que** la pièce-support (2) de l'élément de filtration (1) de la partie arrière (108B) est en polyamide.

3. Guide-embout selon l'une des revendications précédentes, **caractérisé en ce que** la pièce-support (2) de l'élément de filtration (1) de la partie arrière (108B) comprend plusieurs ouvertures latérales (5), chaque ouverture latérale (5) comprenant une membrane de filtration (6) de gaz, de préférence la pièce-support (2) comprend de 2 à 4 ouvertures latérales (5).

4. Guide-embout selon l'une des revendications 1 ou 3, **caractérisé en ce que** les membranes de filtration (6) sont formées d'une pièce unique de forme cylindrique en toile en polymère.

5. Guide-embout selon la revendication 4, **caractérisé en ce que** les membranes de filtration (6) sont en polyamide.

6. Guide-embout selon la revendication 1, **caractérisé en ce que** les moyens de solidarisation (212) réciproques comprennent un taraudage et un filetage coopérant l'un avec l'autre, le filetage étant agencé dans la partie avant (108A) et le taraudage étant agencé dans la partie arrière (108B), ou inversement.

7. Guide-embout selon la revendication 1, **caractérisé en ce que** les moyens de fixation (130) de la partie avant (108A) du guide-embout (108) configurés pour permettre une fixation détachable du guide-embout (108) au sein d'une prise de distribution de fluide (100) comprennent un filetage périphérique externe destiné à venir coopérer avec un taraudage aménagé dans le corps (102) de la prise distribution de fluide (100).

8. Guide-embout selon la revendication 1, **caractérisé en ce que** la tête de raccordement (210) de la partie avant (108A) est en acier inoxydable ou en laiton, de préférence en acier inoxydable ou en laiton revêtu d'un revêtement métallique externe, notamment de chrome ou de nickel.

9. Prise de distribution de fluide (100) comprenant un corps de prise (102) dans lequel est agencé un guide-embout (108) selon l'une des revendications précédentes.

10. Utilisation d'une prise de distribution de fluide (100) selon la revendication 9 pour distribuer un gaz ou mélange gazeux, ou du vide au sein d'un bâtiment hospitalier.

## Patentansprüche

1. Endstückführung (108) für einen Fluidverteilungsanschluss (100), umfassend einen vorderen Teil (108A) und einen hinteren Teil (108B), die miteinander verbunden sind, wobei:
- der hintere Teil (108B) ein Filterelement (1) umfasst, das ein Stützteil (2) umfasst, das eine Umfangswand (3) umfasst, die eine innere Aufnahme (7) begrenzt und sich zwischen einem offenen Ende (2a), das eine Öffnung (4) umfasst, und einem geschlossenen Ende (2b) erstreckt, wobei die Öffnung (4) mit der inneren Aufnahme (7) in Verbindung steht, und wobei die Umfangswand (3) ferner mindestens eine seitliche Öffnung (5) umfasst, die mit der inneren Aufnahme (7) in Verbindung steht, wobei mindestens eine Gasfiltermembran (6) in der mindestens einen seitlichen Öffnung (5) angeordnet ist, so dass sie die mindestens eine seitliche Öffnung (5) abdeckt, und
- der vordere Teil (108A) einen Anschlusskopf (210) umfasst, der einen axialen Gasdurchlass (211) umfasst, der in Fluidverbindung mit der inneren Aufnahme (7) des Gasfilterelements (1) steht, und Befestigungsmittel (130), die dazu ausgestaltet sind, eine lösbare Befestigung der Endstückführung (108) innerhalb eines Fluidverteilungsanschlusses (100) zu ermöglichen, und
- der vordere Teil (108A) und der hintere Teil (108B) ferner wechselseitige Verbindungsmittel (212) umfassen, die es ermöglichen, eine lösbare Befestigung des vorderen Teils (108A) und des hinteren Teils (108B) miteinander zu gewährleisten,
**dadurch gekennzeichnet, dass**:
- das Stützteil (2) des Filterelements (1) des hinteren Teils (108B) aus Polymerwerkstoff ist und
- der Anschlusskopf (210) des vorderen Teils (108A) aus metallischem Werkstoff ist.

2. Endstückführung nach Anspruch 1, **dadurch gekennzeichnet, dass** das Stützteil (2) des Filterelements (1) des hinteren Teils (108B) aus Polyamid ist.

3. Endstückführung nach einem der vorhergehenden Ansprüche, **dadurch gekennzeichnet, dass** das Stützteil (2) des Filterelements (1) des hinteren Teils (108B) mehrere seitliche Öffnungen (5) umfasst, wobei jede seitliche Öffnung (5) eine Gasfiltermembran (6) umfasst, das Stützteil (2) bevorzugt 2 bis 4 seitliche Öffnungen (5) umfasst.

4. Endstückführung nach einem der Ansprüche 1 oder 3, **dadurch gekennzeichnet, dass** die Filtermembranen (6) aus einem einzigen Stück von zylindrischer Form aus Polymerstoff gebildet sind.

5. Endstückführung nach Anspruch 4, **dadurch gekennzeichnet, dass** die Filtermembranen (6) aus Polyamid sind.

6. Endstückführung nach Anspruch 1, **dadurch gekennzeichnet, dass** die wechselseitigen Verbindungsmittel (212) ein Innengewinde und ein Außengewinde umfassen, die miteinander zusammenwirken, wobei das Außengewinde in dem vorderen Teil (108A) angeordnet ist und wobei das Innengewinde in dem hinteren Teil (108B) angeordnet ist oder umgekehrt.

7. Endstückführung nach Anspruch 1, **dadurch gekennzeichnet, dass** die Befestigungsmittel (130) des vorderen Teils (108A) der Endstückführung (108), die dazu ausgestaltet sind, eine lösbare Befestigung der Endstückführung (108) innerhalb eines Fluidverteilungsanschlusses (100) zu ermöglichen, ein äußeres umfängliches Außengewinde umfassen, das dazu bestimmt ist, mit einem Innengewinde zusammenzuwirken, das in dem Körper (102) des Fluidverteilungsanschlusses (100) angeordnet ist.

8. Endstückführung nach Anspruch 1, **dadurch gekennzeichnet, dass** der Anschlusskopf (210) des vorderen Teils (108A) aus nichtrostendem Stahl oder aus Messing ist, bevorzugt aus nichtrostendem Stahl oder aus Messing, der bzw. das mit einer metallischen Außenbeschichtung beschichtet ist, insbesondere mit Chrom oder Nickel.

9. Fluidverteilungsanschluss (100), umfassend einen Anschlusskörper (102), in dem eine Endstückführung (108) nach einem der vorhergehenden Ansprüche angeordnet ist.

10. Verwendung eines Fluidverteilungsanschlusses (100) nach Anspruch 9 zum Verteilen eines Gases oder Gasgemisches oder von Vakuum in einem Krankenhausgebäude.

## Claims

1. Tip guide (108) for a fluid distribution outlet (100), comprising a front part (108A) and a rear part (108B) that are secured to one another, wherein:
- the rear part (108B) comprises a filtration element (1) comprising a support component (2) comprising a peripheral wall (3) delimiting an internal housing (7) and extending between an open end (2a) comprising an orifice (4) and a blind end (2b), the orifice (4) communicating with the internal housing (7), and the peripheral wall (3) also comprising at least one lateral opening (5) communicating with the internal housing (7), at least one gas filtration membrane (6) being arranged in said at least one lateral opening (5) so as to cover said at least one lateral opening (5), and
- the front part (108A) comprises a connection head (210) comprising an axial gas passage (211) in fluidic communication with the internal housing (7) of the gas filtration element (1), and fastening means (130) configured to allow detachable fastening of the tip guide (108) within a fluid distribution outlet (100), and
- the front part (108A) and the rear part (108B) also comprise reciprocal securing means (212) that make it possible to ensure detachable fastening of said front part (108A) and said rear part (108B) to one another,
**characterized in that**:
- the support component (2) of the filtration element (1) of the rear part (108B) is made of a polymer material, and
- the connection head (210) of the front part (108A) is made of a metallic material.

2. Tip guide according to Claim 1, **characterized in that** the support component (2) of the filtration element (1) of the rear part (108B) is made of polyamide.

3. Tip guide according to either of the preceding claims, **characterized in that** the support component (2) of the filtration element (1) of the rear part (108B) comprises a plurality of lateral openings (5), each lateral opening (5) comprising a gas filtration membrane (6), preferably the support component (2) comprises from two to four lateral openings (5).

4. Tip guide according to either of Claims 1 and 3, **characterized in that** the filtration membranes (6) are formed of a single piece of cylindrical shape made of polymer fabric.

5. Tip guide according to Claim 4, **characterized in that** the filtration membranes (6) are made of polyamide.

6. Tip guide according to Claim 1, **characterized in that** the reciprocal securing means (212) comprise a tapped portion and a threaded portion that cooperate with one another, the threaded portion being arranged in the front part (108A) and the tapped portion being arranged in the rear part (108B), or vice versa.

7. Tip guide according to Claim 1, **characterized in that** the fastening means (130) of the front part (108A) of the tip guide (108) that are configured to allow detachable fastening of the tip guide (108) within a fluid distribution outlet (100) comprise an external peripheral threaded portion intended to cooperate with a tapped portion provided in the body (102) of the fluid distribution outlet (100).

8. Tip guide according to Claim 1, **characterized in that** the connection head (210) of the front part (108A) is made of stainless steel or brass, preferably stainless steel or brass coated with an external metallic coating, in particular of chromium or nickel.

9. Fluid distribution outlet (100) comprising an outlet body (102) in which a tip guide (108) according to one of the preceding claims is arranged.

10. Use of a fluid distribution outlet (100) according to Claim 9 for distributing a gas or gaseous mixture or vacuum within a hospital building.
